# EUROPEAN PATENT APPLICATION

(11) **EP 4 775 122 A1**
(43) Date of publication of application: **15.07.2026**
(21) Application number: 25151167.1
(22) Date of filing: 10.01.2025
(51) Int. Cl.: A61B 1/00, A61B 1/05, G02B 23/24, H04N 23/50

(54) **PIPELINE ENDOSCOPE PROBE**

(71) Applicant: Shenzhen Huatuo Global Trade Co., Ltd., Shenzhen (CN)
(72) Inventor: ZHONG, Qianhua, Yichun City, Jiangxi Province (CN)
(74) Representative: Metida

(57) **Abstract**

The present disclosure discloses a pipeline endoscope probe, including an image acquisition device. The image acquisition device includes a lens and a first control panel. The lens is configured to acquire an image. The first control panel receives the image acquired by the lens and converts the image into an electrical signal. The pipeline endoscope probe includes an electrical connection device. The electrical connection device includes an accommodating shell, a spring arranged inside the accommodating shell, a sliding base resisting against the spring, and an ejector pin fixedly connected to the sliding base. The lens can acquire an image, and an imaging element on the first control panel converts the image into an electrical signal and transmits the electrical signal. The electrical connection device is provided with the spring and the ejector pin.

## Description

### TECHNICAL FIELD

The present disclosure relates to the technical field of pipeline endoscopes, and in particular, to a pipeline endoscope probe.

### BACKGROUND OF THE INVENTION

Pipelines, as economical, efficient, and safe material transportation products, have always been paid attention to. At present, pipeline endoscopes on the market can be used for internal inspection and observation of pipelines that are at a high temperature, toxic, and radioactive, and cannot be directly observed with human eyes, such as, to automobiles, aviation engines, pipelines, and mechanical parts. The pipeline endoscope can achieve non-destructive testing without removal or damaging the assembly and shutting down equipment, and can be used for video detection on internal welds, corrosion, blockage, differences, foreign matters, and the like in ventilation ducts, air conditioner ducts, water pipes, and industrial pipelines.

When an endoscope is used to observe and detect a situation inside a pipeline, an electrically connected position at a tail end is easily loosened when an endoscope probe extends into the pipeline, resulting in poor contact and unclear image information acquired by an image acquisition device, and even missing image information acquired by the image acquisition device.

Therefore, the present disclosure provides a pipeline endoscopic probe with a spring electrical-connection device, which can effectively solve the above-mentioned problems.

### SUMMARY OF THE INVENTION

To overcome the shortcomings in the prior art, the present disclosure provides a pipeline endoscope probe which has a simple structure and can further reduce the risk of poor contact caused by looseness of an electrical connection position and effectively ensure the user experience, thereby further improving the detection efficiency and accuracy.

The technical solution adopted by the present disclosure to solve the technical problem is as follows.

A pipeline endoscopic probe, includes:
an image acquisition device, wherein the image acquisition device includes a lens and a first control panel; the lens is configured to acquire an image; the first control panel receives the image acquired by the lens and converts the image into an electrical signal;
an electrical connection device, wherein the image acquisition device is electrically connected to the electrical connection device; the electrical connection device includes an accommodating shell, a spring, a sliding base, and an ejector pin; the spring is arranged inside the accommodating shell; the sliding base resists against the spring; the ejector pin is fixedly connected to the sliding base; and the sliding base is slidably arranged in the accommodating shell.

As an improvement of the present disclosure, the accommodating shell includes a mounting end, a resisting end, and a connection through hole; the connection through hole is communicated to the mounting end and the resisting end; the mounting end is opposite to the resisting end; and the spring is arranged in the connection through hole.

As an improvement of the present disclosure, the mounting end is provided with a mounting opening; the resisting end is provided with a resisting opening; the mounting opening corresponds to an external dimension of the sliding base; the resisting opening corresponds to an external dimension of the ejector pin; and the external dimension of the sliding base is larger than the external dimension of the ejector pin.

As an improvement of the present disclosure, the sliding base includes a first resisting portion; the resisting end is provided with a second resisting portion; a tail end of the second resisting portion forms the resisting opening; and the first resisting portion resists against the second resisting portion.

As an improvement of the present disclosure, the sliding base further includes a third resisting portion, and the third resisting portion resists against the spring.

As an improvement of the present disclosure, the pipeline endoscope probe further includes a mounting base, the mounting base includes a first mounting portion; the mounting end is provided with a second mounting portion; and the first mounting portion and the second mounting portion are fixedly connected to each other.

As an improvement of the present disclosure, the pipeline endoscope probe further includes a mounting shell, the mounting shell includes a first mounting shell and a second mounting shell; the first mounting shell and the second mounting shell are fixedly connected to each other; the first mounting shell and the second mounting shell jointly define an accommodating chamber; and the accommodating chamber is configured to accommodate the mounting base and the electrical connection device.

As an improvement of the present disclosure, the first mounting shell is provided with a first threaded portion; the second mounting shell is provided with a second threaded portion; and the first mounting shell and the second mounting shell are in threaded connection through the first threaded portion and the second threaded portion.

As an improvement of the present disclosure, the pipeline endoscope probe further includes a sealing ring, the sealing ring is arranged between the first mounting shell and the second mounting shell.

As an improvement of the present disclosure, the pipeline endoscope probe further includes a receiving shell and an eccentric member, the receiving shell is provided with a receiving chamber and a receiving through hole communicated to the receiving chamber; the eccentric member is rotatably arranged in the receiving chamber; the image acquisition device is arranged in the accommodating chamber and faces the receiving through hole; the image acquisition device is fixedly connected to the eccentric member; and the image acquisition device is coaxial with the eccentric member and rotates around an axis of the image acquisition device under the action of the eccentric member.

As an improvement of the present disclosure, the eccentric member is provided with a mounting slot and a connection block; at least a portion of the image acquisition device is inserted into the mounting slot to cause the image acquisition device to be coaxial with the eccentric member; and the connection block is configured to connect a connector that passes through the image acquisition device.

As an improvement of the present disclosure, the pipeline endoscope probe further includes an eccentric member shell and a bearing, the eccentric member shell is arranged in the accommodating chamber; the bearing is inserted into the eccentric member shell; and the eccentric member is rotatably inserted into a bearing hole of the bearing.

As an improvement of the present disclosure, the pipeline endoscope probe further includes a first fixing member, the first fixing member is connected to one end of the eccentric member shell close to the bearing; and the first fixing member is configured to fix the bearing in the eccentric member shell.

As an improvement of the present disclosure, a limiting boss is arranged on an inner wall of the eccentric member shell; and the limiting boss resists against the bearing to fix the bearing between the limiting boss and the first fixing member.

As an improvement of the present disclosure, the pipeline endoscope probe further includes an illumination device, the illumination device is arranged at a position of the receiving shell close to the receiving through hole; and an illumination direction of the illumination device is matched with an orientation of the image acquisition device.

As an improvement of the present disclosure, the illumination device includes a plurality of light beads; a light bead slot is provided in a position of the receiving shell surrounding the receiving through hole; and the light beads are arranged in the light bead slot.

As an improvement of the present disclosure, the illumination device further includes a lampshade; and the lampshade is connected to the accommodating shell and covers the light bead slot and the receiving through hole.

As an improvement of the present disclosure, the pipeline endoscope probe further includes a second control panel and a first electrical connection wire, the second control panel is arranged in the accommodating chamber; and the second control panel is electrically connected to the illumination device through the first electrical connection wire.

As an improvement of the present disclosure, the pipeline endoscope probe further includes a second fixing member, the second fixing member is connected to an inner wall of the accommodating chamber and resists against the second control panel, so that the second control panel is defined between the first fixing member and the second fixing member.

As an improvement of the present disclosure, the pipeline endoscope probe further includes a second electrical connection wire, the eccentric member is provided with a wiring hole on one side close to the mounting slot; and the second electrical connection wire passes through the wiring hole to achieve electrical connection between the first control panel and the second control panel.

Beneficial effects: By the arrangement of the above structure, during use, the lens can acquire an image, and an imaging element on the first control panel converts the image into an electrical signal and transmits the electrical signal. The electrical connection device is provided with the spring and the ejector pin. The presence of the spring can provide a pretightening force to the ejector pin of the electrical connection device, so that the ejector pin of the electrical connection device tightly presses against an electrical connection position, thereby ensuring the stability of electrical connection and lowering the risk of poor contact caused by looseness of the electrical connection position. This ensures a stable process of converting, by the imaging element on the first control panel, the image into the electrical signal and transmitting the electrical signal, thereby ensuring the clarity and completeness of the image and further enhancing the user experience.

### BRIEF DESCRIPTION OF DRAWINGS

In order to explain the technical solutions of the embodiments of the present disclosure more clearly, the following will briefly introduce the accompanying drawings used in the embodiments. The drawings in the following description are only some embodiments of the present disclosure. Those of ordinary skill in the art can obtain other drawings based on these drawings without creative work. In addition, the accompanying drawings are not drawn to a scale of 1:1, and the relative dimensions of the various elements are only shown as examples in the diagrams, not necessarily drawn to a true scale.

The present disclosure is further described below in detail in combination with the accompanying drawings and embodiments.
FIG. 1 is a schematic diagram of an overall structure of a pipeline endoscope probe according to the present disclosure;
FIG. 2 is a schematic exploded diagram of a pipeline endoscope probe in a first angle according to the present disclosure;
FIG. 3 is a schematic exploded diagram of a pipeline endoscope probe in a second angle according to the present disclosure;
FIG. 4 is an enlarged view of circle A in FIG. 2;
FIG. 5 is an enlarged view of circle B in FIG. 3;
FIG. 6 is a schematic exploded diagram of an electrical connection device 200 of a pipeline endoscope probe in a first angle according to the present disclosure;
FIG. 7 is a schematic exploded diagram of an electrical connection device 200 of a pipeline endoscope probe in a second angle according to the present disclosure;
FIG. 8 is a schematic cross-sectional structural diagram of an electrical connection device 200 of a pipeline endoscope probe according to the present disclosure; and
FIG. 9 is a schematic enlarged diagram of a partial structure of a pipeline endoscope probe according to the present disclosure.

### DETAILED DESCRIPTION OF THE INVENTION

To make the aforementioned objectives, features, and advantages of the present disclosure more comprehensible, specific implementations of the present disclosure are described in detail below in conjunction with the accompanying drawings. In the following description, numerous specific details are set forth to provide a thorough understanding of the present disclosure. The present disclosure may, however, be embodied in many forms different from that described here. A person skilled in the art can make similar improvements without departing from the connotation of the present disclosure. Therefore, the present disclosure is not limited by the specific embodiments disclosed below.

In the description of the present disclosure, It is to be understood that, The terms "center", "longitudinal", "transverse", "upper", "lower", "front", "rear", "left", "right", "vertical", "horizontal", "top", "bottom", "inner", "outer", "clockwise", "counterclockwise", and the like indicate azimuth or positional relationships based on the azimuth or positional relationships shown in the drawings, For purposes of convenience only of describing the present disclosure and simplifying the description, Rather than indicating or implying that the indicated device or element must have a particular orientation, be constructed and operated in a particular orientation, therefore, not to be construed as limiting the present disclosure.

In addition, the terms "first" and "second" are used for descriptive purposes only, while not to be construed as indicating or implying relative importance or implicitly specifying the number of technical features indicated thereby, features defining "first," "second," and "second" may explicitly or implicitly include one or more of the described features. In the description of the present disclosure, "multiple" means two or more unless explicitly specified otherwise.

In addition, the terms "install", "arrange", "provide", "connect" and "couple" should be understood broadly. For example, it can be a fixed connection, a detachable connection, an integral structure, a mechanical connection, an electrical connection, a direct connection, an indirect connection through an intermediate medium, or a communication between two devices, elements or components. For ordinary technical personnel in this field, the specific meanings of the above terms in present disclosure can be understood based on specific circumstances.

In the present disclosure, unless specific regulation and limitation otherwise, the first feature "onto" or "under" the second feature may include the direct contact of the first feature and the second feature, or may include the contact of the first feature and the second feature through other features between them instead of direct contact. Moreover, the first feature "onto", "above" and "on" the second feature includes that the first feature is right above and obliquely above the second feature, or merely indicates that the horizontal height of the first feature is higher than the second feature. The first feature "under", "below" and "down" the second feature includes that the first feature is right above and obliquely above the second feature, or merely indicates that the horizontal height of the first feature is less than the second feature.

It should be noted that when an element is referred to as being "fixed to" another element, the element can be directly on another component or there can be a centered element. When an element is considered to be "connected" to another element, the element can be directly connected to another element or there may be a centered element. The terms "inner", "outer", "left", "right", and similar expressions used herein are for illustrative purposes only and do not necessarily represent the only implementation.

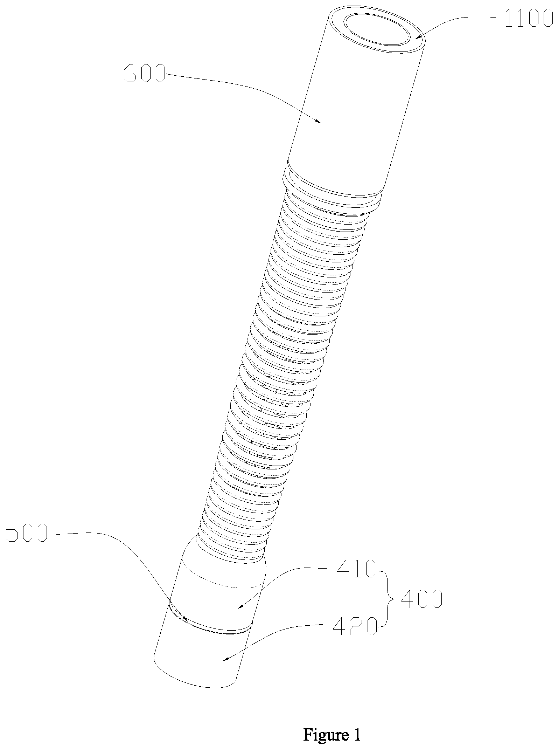

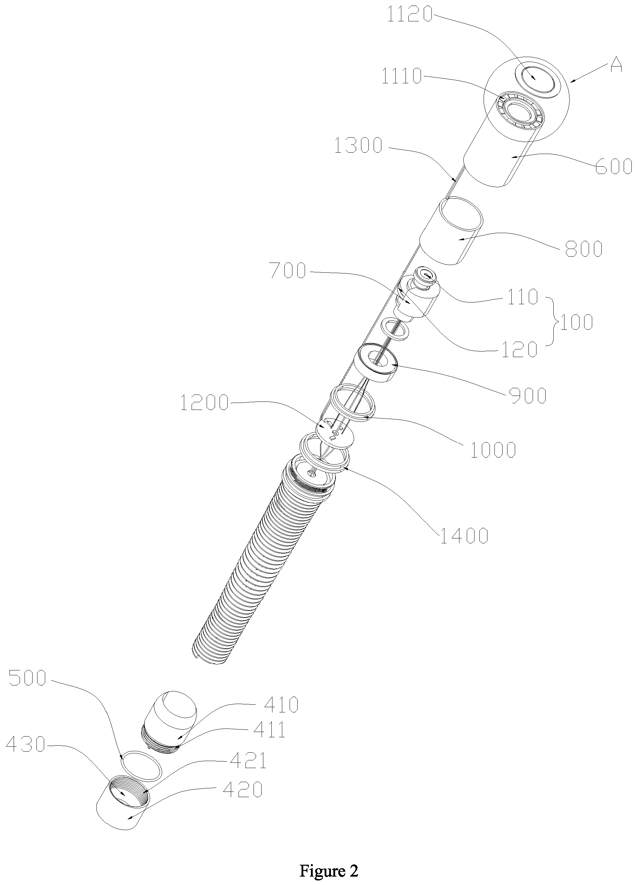

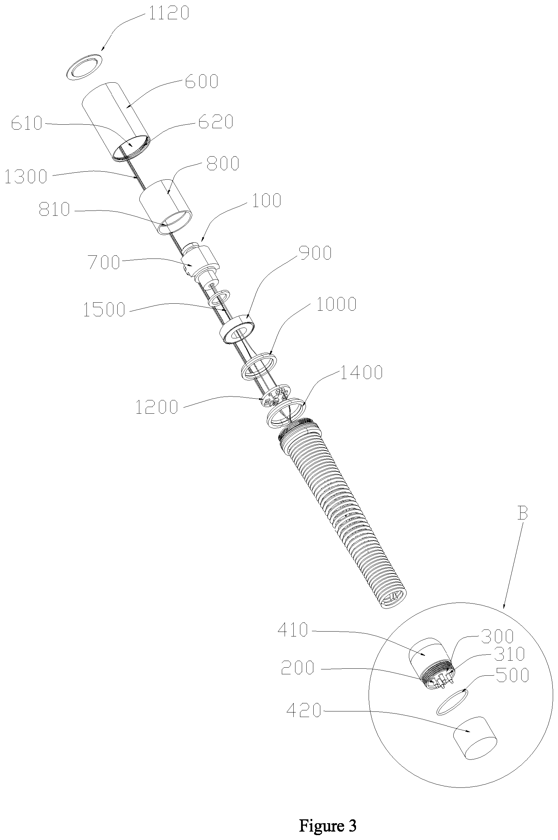

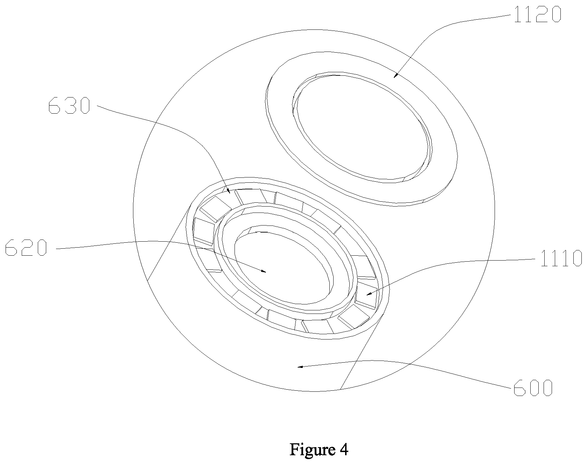

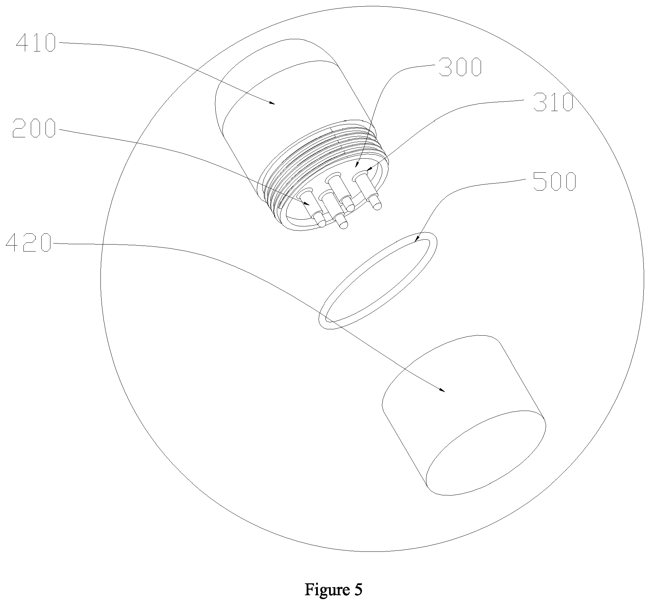

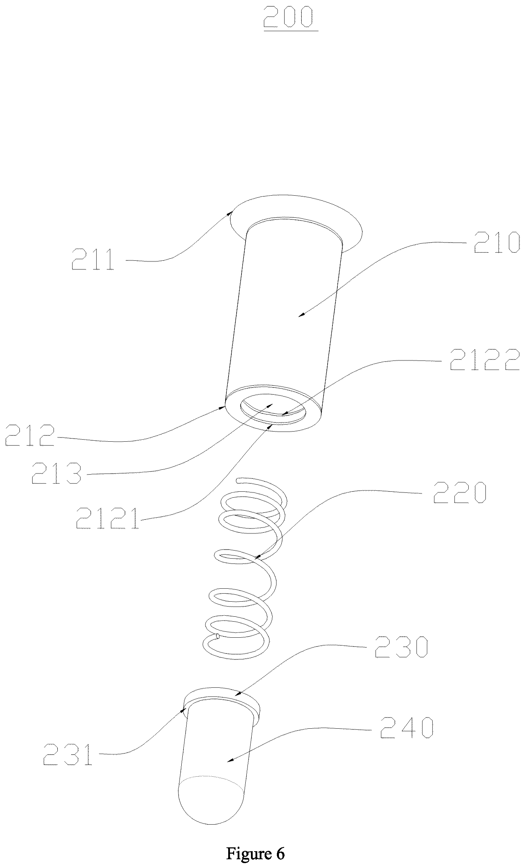

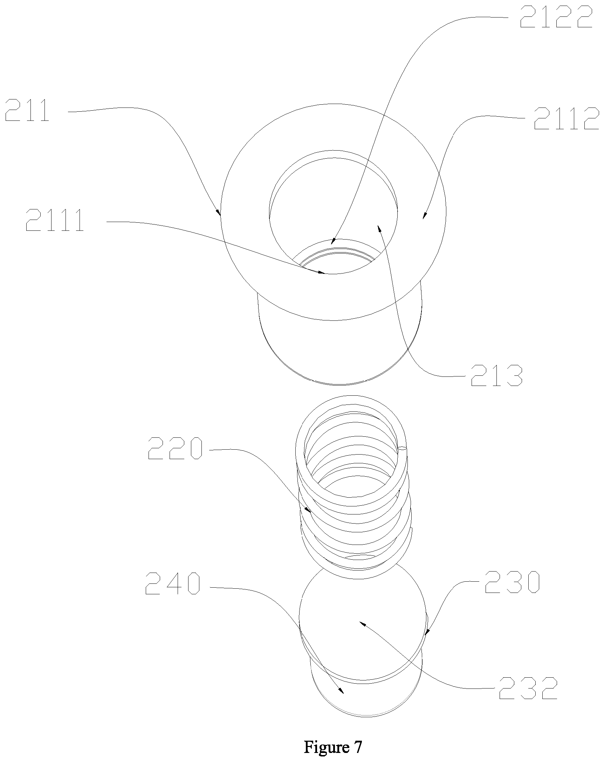

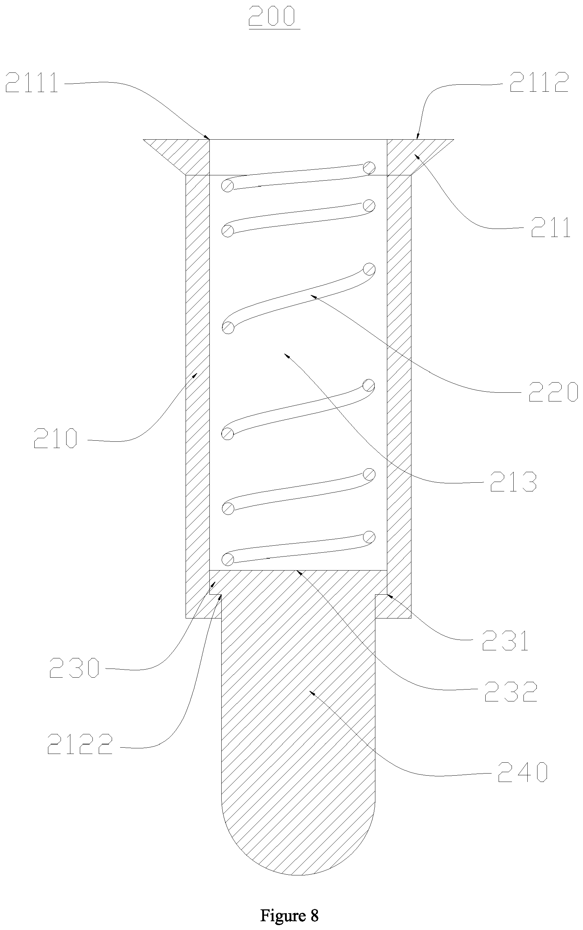

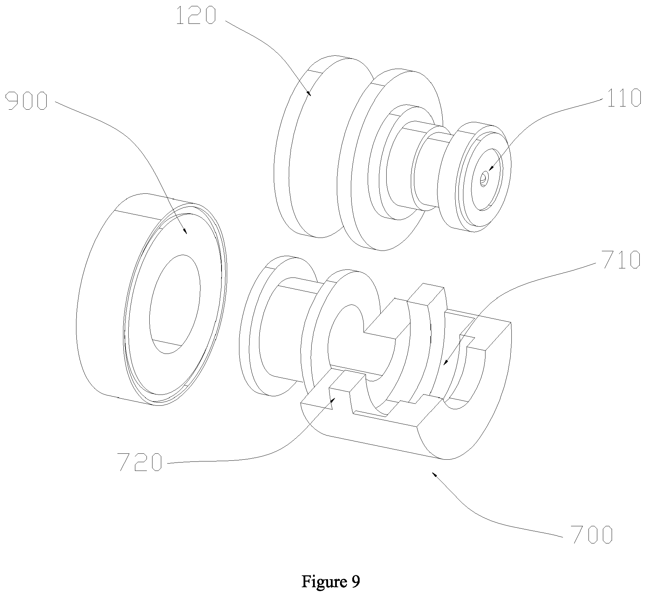

Referring to FIG. 1 to FIG. 9, a pipeline endoscope probe includes:
an image acquisition device 100, wherein the image acquisition device 100 includes a lens 110 and a first control panel 120; the lens 110 is configured to acquire an image; the first control panel 120 receives the image acquired by the lens 110 and converts the image into an electrical signal;
an electrical connection device 200, wherein the image acquisition device 100 is electrically connected to the electrical connection device 200; the electrical connection device 200 includes an accommodating shell 210, a spring 220, a sliding base 230, and an ejector pin 240; the spring 220 is arranged inside the accommodating shell 210; the sliding base 230 resists against the spring 220; the ejector pin 240 is fixedly connected to the sliding base 230; and the sliding base 230 is slidably arranged in the accommodating shell 210.

By the arrangement of the above structure, during use, the lens 110 can acquire an image, and an imaging element on the first control panel 120 converts the image into an electrical signal and transmits the electrical signal. The electrical connection device 200 is provided with the spring 220 and the ejector pin 240. The presence of the spring 220 can provide a pretightening force to the ejector pin 240 of the electrical connection device 200, so that the ejector pin 240 of the electrical connection device tightly presses against an electrical connection position, thereby ensuring the stability of electrical connection and lowering the risk of poor contact caused by looseness of the electrical connection position. This ensures a stable process of converting, by the imaging element on the first control panel 120, the image into the electrical signal and transmitting the electrical signal, thereby ensuring the clarity and completeness of the image and further enhancing the user experience.

In this embodiment, the accommodating shell 210 includes a mounting end 211, a resisting end 212, and a connection through hole 213; the connection through hole 213 is communicated to the mounting end 211 and the resisting end 212; the mounting end 211 is opposite to the resisting end 212; and the spring 220 is arranged in the connection through hole 213. By the arrangement of the above structure, during use, the sliding base 230 and the ejector pin 240 fixedly connected to the sliding base 230 are mounted to the resisting end 212 of the accommodating shell 210 through the mounting end 211 of the accommodating shell 210, and then the spring 220 is placed into the connection through hole 213 of the accommodating shell 210, so that in the electrical connection process, the ejector pin 240 is always pressed by the pretightening force provided by the spring, which ensures that the ejector pin 240 firmly resists against the electrical connection position to ensure the stability of electrical connection. This ensures the clarity and completeness of the image and further enhances the user experience.

In this embodiment, the mounting end 211 is provided with a mounting opening 2111; the resisting end 212 is provided with a resisting opening 2121; the mounting opening 2111 corresponds to an external dimension of the sliding base 230; the resisting opening 2121 corresponds to an external dimension of the ejector pin 240; and the external dimension of the sliding base 230 is larger than the external dimension of the ejector pin 240. By the arrangement of the above structure, during use, the sliding base 230 and the ejector pin 240 fixedly connected to the sliding base 230 are mounted to the resisting opening 2121 of the resisting end 212 through the mounting opening 2111 of the mounting end 211, and then the spring 220 is mounted into the accommodating shell 210, so that in the electrical connection process, the ejector pin 240 is always pressed by the pretightening force provided by the spring, which ensures that the ejector pin 240 firmly resists against the electrical connection position. At the same time, the mounting opening 2111 corresponds to the external dimension of the sliding base 230; the resisting opening 2121 corresponds to the external dimension of the ejector pin 240; and the external dimension of the sliding base 230 is larger than the external dimension of the ejector pin 240. During maintenance, after the resisting state is relieved, the ejector pin 240, which is subjected to the pretightening force, will not pop out of the resisting opening 2121 of the resisting end 212 and will not be detached from the accommodating shell 210. This improves the maintenance efficiency and further enhances the user experience.

In this embodiment, the sliding base 230 includes a first resisting portion 231; the resisting end 212 is provided with a second resisting portion 2122; a tail end of the second resisting portion 2122 forms the resisting opening 2121; and the first resisting portion 231 resists against the second resisting portion 2122. By the arrangement of the above structure, during use, the first resisting portion 231 of the sliding base 230 resists against the resisting opening 2121 formed at the tail end of the second resisting portion 2122. During maintenance, after the resisting state of the ejector pin 240 which is subjected to the pretightening force is relieved, the second resisting portion 2122 surrounding the resisting opening 2121 prevents the ejector pin 240 fixedly connected to the sliding base 230 from popping out and being detached from the accommodating shell 210, thereby improving the maintenance efficiency and further enhancing the user experience.

In this embodiment, the sliding base 230 further includes a third resisting portion 232, and the third resisting portion 232 resists against the spring 220. By the arrangement of the above structure, during use, the third resisting portion 232 of the sliding base 230 resists against the spring 220, and the spring 220 is in a compressed state, so that the sliding base 230 is subjected to an outward elastic force. The sliding base 230 transmits the elastic force to the ejector pin 240, ensuring that the ejector pin 240 firmly resists against the electrical connection position, to ensure a stable process of converting, by the imaging element on the first control panel 120, the image into the electrical signal and transmitting the electrical signal. This ensures the clarity and completeness of the image and further enhances the user experience.

In this embodiment, the pipeline endoscope probe further includes a mounting base 300; the mounting base 300 includes a first mounting portion 310; the mounting end 211 is provided with a second mounting portion 2112; and the first mounting portion 310 and the second mounting portion 2112 are fixedly connected to each other. By the arrangement of the above structure, during use, due to the fixed connection between the first mounting portion 310 and the second mounting portion 2112, the electrical connection device 200 will not shake relative to the mounting base 300. This ensures the stability of an image acquisition process of the image acquisition device 100, thereby ensuring the clarity and completeness of a final image, and further enhancing the user experience.

In this embodiment, the pipeline endoscope probe further includes a mounting shell 400; the mounting shell 400 includes a first mounting shell 410 and a second mounting shell 420; the first mounting shell 410 and the second mounting shell 420 are fixedly connected to each other; the first mounting shell 410 and the second mounting shell 420 jointly define an accommodating chamber 430; and the accommodating chamber 430 is configured to accommodate the mounting base 300 and the electrical connection device 200. By the arrangement of the above structure, during use, the mounting base 300 and the electrical connection device 200 are arranged in the accommodating chamber 430 defined by the mounting shell 400. The mounting shell 400 can protect the mounting base 300 and the electrical connection device 200, to reduce the impact of external environmental factors on the mounting base 300 and the electrical connection device 200. Due to the fixed connection between the mounting base 300 and the mounting shell 400, and the fixed connection between the mounting base 300 and the electrical connection device 200, the electrical connection device 200 will not shake relative to the mounting shell 400. This ensures the stability of an image acquisition process when the mounting shell 400 shakes, thereby ensuring the clarity and completeness of a final image and further enhancing the user experience.

In this embodiment, the first mounting shell 410 is provided with a first threaded portion 411; the second mounting shell 420 is provided with a second threaded portion 421; and the first mounting shell 410 and the second mounting shell 420 are in threaded connection through the first threaded portion 411 and the second threaded portion 421. By the arrangement of the above structure, during use, the first mounting shell 410 and the second mounting shell 420 are in threaded connection and tightly combined through the first threaded portion 411 and the second threaded portion 421. During maintenance, it is easy for disassembling to observe an internal situation of the accommodating chamber 430 defined by the first mounting shell 410 and the second mounting shell 420, which improves the maintenance efficiency and further enhances the user experience.

In this embodiment, the pipeline endoscope probe further includes a sealing ring 500 arranged between the first mounting shell 410 and the second mounting shell 420. By the arrangement of the above structure, during use, the sealing ring 500 is arranged between the first mounting shell 410 and the second mounting shell 420, to further improve the airtightness of the accommodating chamber 430, better protect the mounting base 300 and the electrical connection device 200, and prolong their service lives.

In this embodiment, the pipeline endoscope probe further includes a receiving shell 600 and an eccentric member 700; the receiving shell 600 is provided with a receiving chamber 610 and a receiving through hole 620 communicated to the receiving chamber 610; the eccentric member 700 is rotatably arranged in the receiving chamber 610; the image acquisition device 100 is arranged in the accommodating chamber 430 and faces the receiving through hole 620; the image acquisition device 100 is fixedly connected to the eccentric member 700; and the image acquisition device 100 is coaxial with the eccentric member 700 and rotates around an axis of the image acquisition device 100 under the action of the eccentric member 700. By the arrangement of the above structure, during use, the pipeline endoscope probe is inserted into a pipeline, and the eccentric member 700 rotates under the action of the gravity, thereby driving the image acquisition device 100 to rotate, so that an angle of view of the lens 110 of the image acquisition device 100 is always forward, which prevents the image acquisition device 100 from rotating with the pipeline endoscope probe when the pipeline endoscope probe is inserted into the pipeline. This maintains the stability of the image acquired by the image acquisition device 100 and allows a user to better understand a situation inside the pipeline according to information acquired by the image acquisition device.

In this embodiment, the eccentric member 700 is provided with a mounting slot 710 and a connection block 720; at least a portion of the image acquisition device 100 is inserted into the mounting slot 710 to cause the image acquisition device 100 to be coaxial with the eccentric member 700; and the connection block 720 is configured to connect a connector that passes through the image acquisition device 100. By the arrangement of the above structure, the image acquisition device 100 is plugged into the mounting slot 710. On the one hand, it can more stably connect the image acquisition device 100 to the eccentric member 700. On the other hand, it can coaxially arrange the image acquisition device 100 and the eccentric member 700, making the image acquisition device 100 rotate more smoothly with the eccentric member 700. Due to the use of the connection block 720, it can further stably connect the image acquisition device 100 to the eccentric member 700, to prevent the image acquisition device 100 from being detached from the mounting slot 710 and improve the stability of the product.

In this embodiment, the pipeline endoscope probe further includes an eccentric member shell 800 and a bearing 900; the eccentric member shell 800 is arranged in the accommodating chamber 430; the bearing 900 is inserted into the eccentric member shell 800; and the eccentric member 700 is rotatably inserted into a bearing hole of the bearing 900. By the arrangement of the above structure, due to the bearing 900, the eccentric member 700 can rotate more smoothly around a rotation axis, thereby driving the image acquisition device 100 to rotate synchronously and making the product more sensitive. When the pipeline endoscope probe is flipped, the image acquisition device 100 can rotate quickly and flexibly under the action of the gravity, so that the image acquired by the image acquisition device 100 always faces up, making it easier for a user to determine a situation inside a pipeline based on the image acquired by the image acquisition device 100. The eccentric member shell 800 can better protect the image acquisition device 100 and the eccentric member 700, and the eccentric member shell 800, the image acquisition device 100, and the eccentric member 700 form a whole, which facilitates the assembling of the product and improves the production efficiency of the product.

In this embodiment, the pipeline endoscope probe further includes a first fixing member 1000; the first fixing member 1000 is connected to one end of the eccentric member shell 800 close to the bearing 900; and the first fixing member 1000 is configured to fix the bearing 900 in the eccentric member shell 800. By the arrangement of the above structure, the first fixing member 1000 can further fix the bearing 900, to prevent the eccentric member 700 and the image acquisition device 100 from being detached from the eccentric member shell 800, and improve the stability of the product.

In this embodiment, a limiting boss 810 is arranged on an inner wall of the eccentric member shell 800; and the limiting boss 810 resists against the bearing 900 to fix the bearing 900 between the limiting boss 810 and the first fixing member 1000. By the arrangement of the above structure, two ends of the bearing 900 respectively resist against the limiting boss 810 and the first fixing member 1000, which can further fix the bearing 900, hinder an axial movement of the bearing 900, and improve the stability of the product.

In this embodiment, the pipeline endoscope probe further includes an illumination device 1100; the illumination device 1100 is arranged at a position of the receiving shell 600 close to the receiving through hole 620; and an illumination direction of the illumination device 1100 is matched with an orientation of the image acquisition device 100. By the arrangement of the above structure, the illumination device 1100 can provide light, making the image acquired by the image acquisition device 100 clearer, so that a user can better understand the situation inside the pipeline, and the product is used more conveniently. In addition, the illumination direction of the illumination device 1100 is matched with the orientation of the image acquisition device 100, which can effectively illuminate an environment in front of the image acquisition device 100, improve the efficiency and clarity of the image acquired by the image acquisition device 100. Moreover, the image acquisition device 100 can rotate in the receiving chamber 610, so that it is convenient for a user to change the angle of view to learn about the situation inside the pipeline more clearly and intuitively.

In this embodiment, the illumination device 1100 includes a plurality of light beads 1110; a light bead slot 630 is provided in a position of the receiving shell 600 surrounding the receiving through hole 620; and the light beads 1110 are arranged in the light bead slot 630. By the arrangement of the above structure, during use, the light beads 1110 are arranged in the light bead slot 630 and surround the receiving through hole 620, so that light around the receiving through hole 620 can be more uniform, thereby making the light in the environment in front of the image acquisition device 100 more uniform. The image acquired by the image acquisition device 100 is clearer, which prevents the impact of too dark or too bright local light on the clarity of the image.

In this embodiment, the illumination device 1100 further includes a lampshade 1120; and the lampshade 1120 is connected to the accommodating shell 210 and covers the light bead slot 630 and the receiving through hole 620. By the arrangement of the above structure, the lampshade 1120 can effectively isolate the light beads 1110 and the image acquisition device 100 from an external environment during use, which improves the airtightness of the product, prevents debris in the pipeline from damaging the light beads 1110 and the image acquisition device 100, and prolongs the service life of the product. Preferably, the lampshade 1120 is made of transparent high-strength glass, plastic, or resin.

In this embodiment, the pipeline endoscope probe further includes a second control panel 1200 and a first electrical connection wire 1300. The second control panel 1200 is arranged in the accommodating chamber 430; and the second control panel 1200 is electrically connected to the illumination device 1100 through the first electrical connection wire 1300. By the arrangement of the above structure, during use, the second control panel 1200 can control the illumination device 1100 according to a command of a user, such as turning on or turning off some of the light beads 1110, or adjusting the brightness of the light beads 1110. When light inside the pipeline is insufficient, the light beads can effectively supplement the lighting; and when light inside the pipeline is sufficient, some of the light beads 1110 can be turned off to save energy. Meanwhile, adjusting the light intensity can also change the ambient brightness, prevent excessive brightness from causing overexposure of the image acquired by the image acquisition device 100, ensure the clarity of the image acquired by the image acquisition device 100, and help a user better understand the situation inside the pipeline.

In this embodiment, the pipeline endoscope probe further includes a second fixing member 1400; the second fixing member 1400 is connected to an inner wall of the accommodating chamber 430 and resists against the second control panel 1200, so that the second control panel 1200 is defined between the first fixing member 1000 and the second fixing member 1400. By the arrangement of the above structure, the second fixing member 1400 and the first fixing member 1000 fix the second control panel 1200, which further improves the stability of the product and prevents the second control panel 1200 from moving inside the receiving chamber 610. Preferably, the second fixing member 1400 is connected to the inner wall of the receiving chamber 610 through a threaded portion to improve the stability of connection.

In this embodiment, the pipeline endoscope probe further includes a second electrical connection wire 1500; the eccentric member 700 is provided with a wiring hole on one side close to the mounting slot 710; and the second electrical connection wire 1500 passes through the wiring hole to achieve electrical connection between the first control panel 120 and the second control panel 1200. By the arrangement of the above structure, during use, the second electrical connection wire 1500 passes through the wiring hole, ensuring the electrical connection between the first control panel 120 and the second control panel 1200 and avoid impact of the second electrical connection wire 1500 on the rotation of the eccentric member 700. This further improves the stability of the product.

As described above, one or more embodiments are provided in conjunction with the detailed description, The specific implementation of the present disclosure is not confirmed to be limited to that the description is similar to or similar to the method, the structure and the like of the present disclosure, or a plurality of technical deductions or substitutions are made on the premise of the conception of the present disclosure to be regarded as the protection of the present disclosure.

## Claims

1. A pipeline endoscopic probe, comprising:
an image acquisition device, wherein the image acquisition device comprises a lens and a first control panel; the lens is configured to acquire an image; the first control panel receives the image acquired by the lens and converts the image into an electrical signal;
an electrical connection device, wherein the image acquisition device is electrically connected to the electrical connection device; the electrical connection device comprises an accommodating shell, a spring, a sliding base, and an ejector pin; the spring is arranged inside the accommodating shell; the sliding base resists against the spring; the ejector pin is fixedly connected to the sliding base; and the sliding base is slidably arranged in the accommodating shell.

2. The pipeline endoscope probe according to claim 1, wherein the accommodating shell comprises a mounting end, a resisting end, and a connection through hole; the connection through hole is communicated to the mounting end and the resisting end; the mounting end is opposite to the resisting end; and the spring is arranged in the connection through hole.

3. The pipeline endoscope probe according to claim 2, wherein the mounting end is provided with a mounting opening; the resisting end is provided with a resisting opening; the mounting opening corresponds to an external dimension of the sliding base; the resisting opening corresponds to an external dimension of the ejector pin; and the external dimension of the sliding base is larger than the external dimension of the ejector pin.

4. The pipeline endoscope probe according to claim 3, wherein the sliding base comprises a first resisting portion; the resisting end is provided with a second resisting portion; a tail end of the second resisting portion forms the resisting opening; and the first resisting portion resists against the second resisting portion.

5. The pipeline endoscope probe according to claim 3, wherein the sliding base further comprises a third resisting portion, and the third resisting portion resists against the spring.

6. The pipeline endoscope probe according to claim 3, further comprising a mounting base, wherein the mounting base comprises a first mounting portion; the mounting end is provided with a second mounting portion; and the first mounting portion and the second mounting portion are fixedly connected to each other.

7. The pipeline endoscope probe according to claim 6, further comprising a mounting shell, wherein the mounting shell comprises a first mounting shell and a second mounting shell; the first mounting shell and the second mounting shell are fixedly connected to each other; the first mounting shell and the second mounting shell jointly define an accommodating chamber; and the accommodating chamber is configured to accommodate the mounting base and the electrical connection device.

8. The pipeline endoscope probe according to claim 7, wherein the first mounting shell is provided with a first threaded portion; the second mounting shell is provided with a second threaded portion; and the first mounting shell and the second mounting shell are in threaded connection through the first threaded portion and the second threaded portion.

9. The pipeline endoscope probe according to claim 8, further comprising a sealing ring, wherein the sealing ring is arranged between the first mounting shell and the second mounting shell.

10. The pipeline endoscope probe according to claim 1, further comprising a receiving shell and an eccentric member, wherein the receiving shell is provided with a receiving chamber and a receiving through hole communicated to the receiving chamber; the eccentric member is rotatably arranged in the receiving chamber; the image acquisition device is arranged in the accommodating chamber and faces the receiving through hole; the image acquisition device is fixedly connected to the eccentric member; and the image acquisition device is coaxial with the eccentric member and rotates around an axis of the image acquisition device under the action of the eccentric member.

11. The pipeline endoscope probe according to claim 10, wherein the eccentric member is provided with a mounting slot and a connection block; at least a portion of the image acquisition device is inserted into the mounting slot to cause the image acquisition device to be coaxial with the eccentric member; and the connection block is configured to connect a connector that passes through the image acquisition device.

12. The pipeline endoscope probe according to claim 11, further comprising an eccentric member shell and a bearing, wherein the eccentric member shell is arranged in the accommodating chamber; the bearing is inserted into the eccentric member shell; and the eccentric member is rotatably inserted into a bearing hole of the bearing.

13. The pipeline endoscope probe according to claim 12, further comprising a first fixing member, wherein the first fixing member is connected to one end of the eccentric member shell close to the bearing; and the first fixing member is configured to fix the bearing in the eccentric member shell.

14. The pipeline endoscope probe according to claim 13, wherein a limiting boss is arranged on an inner wall of the eccentric member shell; and the limiting boss resists against the bearing to fix the bearing between the limiting boss and the first fixing member.

15. The pipeline endoscope probe according to claim 10, further comprising an illumination device, wherein the illumination device is arranged at a position of the receiving shell close to the receiving through hole; and an illumination direction of the illumination device is matched with an orientation of the image acquisition device.

## Amended claims

### Amended claims in accordance with Rule 137(2) EPC.

1. A pipeline endoscopic probe, comprising:
an image acquisition device (100), wherein the image acquisition device (100) comprises a lens (110) and a first control panel (120); the lens (100) is configured to acquire an image; the first control panel (120) receives the image acquired by the lens (110) and converts the image into an electrical signal;
an electrical connection device (200), wherein the image acquisition device (100) is electrically connected to the electrical connection device (200); the electrical connection device (200) comprises an accommodating shell (210), a spring (220), a sliding base (230), and an ejector pin (240); the spring (220) is arranged inside the accommodating shell (210); the sliding base (230) resists against the spring (220); the ejector pin (240) is fixedly connected to the sliding base (230); and the sliding base (230) is slidably arranged in the accommodating shell (210), **characterized in that**,
the pipeline endoscopic probe further comprises a mounting shell (400) and a sealing ring (500), wherein the mounting shell (400) comprises a first mounting shell (410) and a second mounting shell (420); the first mounting shell (410) is provided with a first threaded portion (411), the second mounting shell (420) is provided with a second threaded portion (421); and the first mounting shell (410) and the second mounting shell (420) are in threaded connection through the first threaded portion (411) and the second threaded portion (421); the first mounting shell (410) and the second mounting shell (420) jointly define an accommodating chamber (430), and the sealing ring (500) is arranged between the first mounting shell (410) and the second mounting shell (420) to seal the accommodating chamber (430);
the accommodating chamber (430) is configured to accommodate the electrical connection device (200), the ejector pin (240) extends in a direction opposite to the image acquisition device (100) and towards the accommodating chamber (430), so that the ejector pin (240) of the electrical connection device (200) tightly presses against an electrical connection position to achieve an electrical connection for the image acquisition device (100); and
the pipeline endoscopic probe further comprises a receiving shell (600) and an eccentric member (700), the receiving shell (600) is provided with a receiving chamber (610) and a receiving through hole (620) communicated to the receiving chamber (610); the eccentric member (700) is rotatably arranged in the receiving chamber (610); the image acquisition device (100) is arranged in the receiving chamber (610) and faces the receiving through hole (620); the image acquisition device (100) is fixedly connected to the eccentric member (700); and the image acquisition device (100) is coaxial with the eccentric member (700) and rotates around an axis of the image acquisition device (100) under the action of the eccentric member (700).

2. The pipeline endoscope probe according to claim 1, wherein the accommodating shell (210) comprises a mounting end (211), a resisting end (212), and a connection through hole (213); the connection through hole (213) is communicated to the mounting end (211) and the resisting end (212); the mounting end (211) is opposite to the resisting end (212); and the spring (220) is arranged in the connection through hole (213).

3. The pipeline endoscope probe according to claim 2, wherein the mounting end (211) is provided with a mounting opening (2111); the resisting end (212) is provided with a resisting opening (2121); the mounting opening (2111) corresponds to an external dimension of the sliding base (230); the resisting opening (2121) corresponds to an external dimension of the ejector pin (240); and the external dimension of the sliding base (230) is larger than the external dimension of the ejector pin (240).

4. The pipeline endoscope probe according to claim 3, wherein the sliding base (230) comprises a first resisting portion (231); the resisting end (212) is provided with a second resisting portion (2122); a tail end of the second resisting portion (2122) forms the resisting opening (2121); and the first resisting portion (231) resists against the second resisting portion (2122).

5. The pipeline endoscope probe according to claim 3, wherein the sliding base (230) further comprises a third resisting portion (232), and the third resisting portion (232) resists against the spring (220).

6. The pipeline endoscope probe according to claim 3, further comprising a mounting base (300) for mounting the electrical connection device (200), and the mounting base (300) is also accommodated in the accommodating chamber (430), wherein the mounting base (300) comprises a first mounting portion (310); the mounting end (211) is provided with a second mounting portion (2112); and the first mounting portion (310) and the second mounting portion (2122) are fixedly connected to each other.

7. The pipeline endoscope probe according to claim 1, wherein the eccentric member (700) is provided with a mounting slot (710) and a connection block (720); at least a portion of the image acquisition device (100) is inserted into the mounting slot (710) to cause the image acquisition device (100) to be coaxial with the eccentric member (700); and the connection block (720) is configured to connect a connector that passes through the image acquisition device (100).

8. The pipeline endoscope probe according to claim 11, further comprising an eccentric member shell (800) and a bearing (900), wherein the eccentric member shell (800) is arranged in the accommodating chamber (430); the bearing (900) is inserted into the eccentric member shell (800); and the eccentric member (700) is rotatably inserted into a bearing hole of the bearing (900).

9. The pipeline endoscope probe according to claim 12, further comprising a first fixing member (1000), wherein the first fixing member (1000) is connected to one end of the eccentric member shell (800) close to the bearing (900); and the first fixing member (10000) is configured to fix the bearing (900) in the eccentric member shell (800).

10. The pipeline endoscope probe according to claim 13, wherein a limiting boss (810) is arranged on an inner wall of the eccentric member shell (800); and the limiting boss (810) resists against the bearing (900) to fix the bearing (900) between the limiting boss (810) and the first fixing member (1000).

11. The pipeline endoscope probe according to claim 1, further comprising an illumination device (1100), wherein the illumination device (1100) is arranged at a position of the receiving shell (600) close to the receiving through hole (620); and an illumination direction of the illumination device (1100) is matched with an orientation of the image acquisition device (100).
